## Europäisches Patentamt

## European Patent Office

## Office européen des brevets

(19)

(11) Veröffentlichungsnummer: **0 082 504**
**B1**

(12)

# EUROPÄISCHE PATENTSCHRIFT

(45) Veröffentlichungstag der Patentschrift:
**04.06.86**

(51) Int. Cl.⁴: **A 61 M 25/00**

(21) Anmeldenummer: **82111740.5**

(22) Anmeldetag: **17.12.82**

(54) **Katheter zur Katheterung zentraler Venen.**

(30) Priorität: **17.12.81 DE 3150052**

(43) Veröffentlichungstag der Anmeldung:
**29.06.83 Patentblatt 83/26**

(45) Bekanntmachung des Hinweises auf die Patenterteilung:
**04.06.86 Patentblatt 86/23**

(84) Benannte Vertragsstaaten:
**AT BE CH DE FR GB IT LI LU NL SE**

(56) Entgegenhaltungen:
**GB - A - 1 208 639**
**US - A - 2 118 631**
**US - A - 3 547 103**
**US - A - 3 757 768**

(73) Patentinhaber: **Sterimed Gesellschaft für medizinischen Bedarf mbH, Fasanerieweg 15-17 Postfach 215, D-6600 Saarbrücken 3 (DE)**

(72) Erfinder: **Kramann, Bernhard, Dr., Preysingstrasse 11, D-8000 München (DE)**
Erfinder: **Rust, Meinhard, Dr., Knappertsbuschstrasse 12, D-8000 München 81 (DE)**

(74) Vertreter: **Suchy, Herbert, Dr., Byk Gulden Lomberg Chemische Fabrik GmbH Byk-Gulden-Strasse 2 Postfach 6500, D-7750 Konstanz (DE)**

ACTORUM AG

## Beschreibung

### Gebiet der Technik

Die Erfindung betrifft einen Katheter zur Katheterung zentraler Venen über periphere Venenzugänge, umfassend einen Katheterschlauch und einen an seinem distalen Ende spazierstockartig gekrümmten Führungsdraht, der gegenüber dem Katheterschlauch verschiebbar ist.

### Stand der Technik

Katheterungen zentraler Venen (Hohlvene, Vena cava) spielen eine wichtige Rolle bei der Schockbehandlung, bei der Bestimmung des zentralvenösen Blutdrucks oder dessen Überwachung bei operativen Eingriffen sowie bei der parenteralen Ernährung. Je nach Eintrittstelle des Katheters in den menschlichen Körper unterscheidet man zwischen den zentralen Zugangswegen und den peripheren Zugangswegen. Im ersten Fall erfolgt der Zugang über die Vena jugularis, die Vena anonyma oder die Vena subclavia, während im zweiten Fall der Zugang über Bein- oder Armvenen erfolgt. Die Katheterung über einen zentralen Zugangsweg hat den Vorteil, dass der Katheter in einem verhältnismässig grosslumigen Gefäss, das keine Venenklappen aufweist, gelegt werden kann und der Weg zur Hohlvene kurz ist. Nachteilig an den zentralen Zugangswegen ist jedoch, dass das Auffinden der für eine Punktion in Frage kommenden Venen schwierig ist und infolge der nahen Nachbarschaft von grossen Arterien besonderer Vorsicht bedarf. So wird die Vena anonyma von der Punktionskanüle erst in ungefähr 5 cm Tiefe erreicht. Zur Punktion der Vena jugularis muss die Punktionskanüle in einem bestimmten Winkel zur Hautfläche durch den Musculus sternocleidomastoideus geführt werden, um in 3 bis 4 cm Tiefe auf die Vene zu stossen, die unmittelbar neben der Arteria carotis verläuft. Die Katheterung von zentralen Venen über einen zentralen Zugang kann daher nur von besonders erfahrenen und geübten Ärzten, wie insbesondere Narkosefachärzten, weitgehend gefahrlos durchgeführt werden. Für weniger Geübte oder bei ungünstigen äusseren Umständen, wie beispielsweise der Versorgung von Unfallopfern am Unfallort oder im Rettungsfahr- oder -flugzeug, ist der Zugang zu den zentralen Venen über eine periphere Vene, also eine Beinvene oder Armvene, vorzuziehen, weil diese Venen meist gut durch die Haut sichtbar sind oder durch kurzes Abbinden des Beines bzw. des Oberarmes leicht auffindbar gemacht werden können. Das Vorschieben des Katheters ist jedoch beim Zugang über eine periphere Vene schwieriger, zum einen, weil der Weg bis zur Hohlvene bedeutend länger ist, zum anderen, weil die peripheren Venen einen kleineren inneren Durchmesser aufweisen und zudem Venenklappen zu überwinden sind.

Daher ergeben sich bei einem erheblichen Prozentsatz der Katheterungen über einen peripheren Zugang Schwierigkeiten beim Vorschieben des Katheterschlauches: Beispielsweise kann die Schlauchspitze einen falschen Weg (Via falsa) einschlagen, an Venenklappen oder Gefässverzweigungen hängenbleiben und lebensbedrohliche Komplikationen, wie Embolien oder Blutungen im Thorax, verursachen. Es besteht daher ein Bedarf nach einer Verbesserung der bisher geübten Methoden der zentralen Venenkatheterung über einen peripheren Zugang und an der Zurverfügungstellung eines entsprechenden Katheters.

Aus der US-A 3 757 768 ist ein Katheter bekannt, dessen Schlauch aus einer Spiralfeder besteht, die von einem Schrumpfschlauch überzogen ist, wobei der Schrumpfschlauch über das distale Ende der Spiralfeder hinausragt. Das Einbringen des Katheters kann durch ein Drahtstilett, dessen distales Ende gebogen gestaltet sein kann, unterstützt werden. Durch Weglassen des Drahtstiletts und Verkürzung des Katheterschlauches auf 2,5 bis 10 cm Länge soll der Katheter auch für die Zwecke der intravenösen Ernährung geeignet sein. Für Katheterungen zentraler Venen über periphere Zugänge ist dieser Katheter jedoch nicht vorgesehen.

Aufgabe der Erfindung war es, einen neuen Katheter für Katheterung zentraler Venen über einen peripheren Zugang, umfassend einen Katheterschlauch und einen an seinem distalen Ende spazierstockartig gekrümmten Führungsdraht, der gegenüber dem Katheterschlauch verschiebbar ist, zur Verfügung zu stellen, der es erlaubt, zentrale Venenkatheterungen, insbesondere auch in Notfallsituationen unter den oft ungünstigen Umgebungsbedingungen eines Unfallortes oder in Rettungsfahr- und -flugzeugen, sicher und schnell durchzuführen.

### Beschreibung der Erfindung

Gegenstand der Erfindung ist ein Katheter zur Katheterung zentraler Venen über periphere Venenzugänge umfassend einen Katheterschlauch und einen an seinem distalen Ende spazierstockartig gekrümmten Führungsdraht, der gegenüber dem Katheterschlauch verschiebbar ist, dadurch gekennzeichnet, dass eine Vorrichtung vorgesehen ist, die die gegenseitige Verschiebbarkeit von Katheterschlauch gegenüber dem Führungsdraht auf ungefähr die Länge des gekrümmten distalen Endes des Führungsdrahtes zu begrenzen erlaubt, wobei in einer Verschiebeendstellung das gekrümmte distale Ende des Führungsdrahtes aus dem distalen Ende des Katheterschlauches herausragt und in der anderen Verschiebeendstellung das gekrümmte distale Ende des Führungsdrahtes vollständig in das distale Ende des Katheterschlauches hineingezogen ist. Unter peripheren Venenzugängen werden insbesondere die Zugänge über die Venen im Bereich der Ellenbeuge und des Handrückens verstanden.

Es wurde nun festgestellt, dass die Katheterung der Vena cava superior über einen peripheren Venenzugang bedeutend besser und ohne Komplikationen mit einem erfindungsgemässen Katheter durchgeführt werden kann. Dieser Befund ist sehr überraschend, da man bisher davon ausging, dass derartige spazierstockartig gekrümmte Mandrins für den Vorschub in Venen nicht geeignet seien, weil sie dort, beispielsweise an den Venenklappen, Verletzungen verursachen würden.

Weitere Ausführungsformen des erfindungsgemässen Katheters ergeben sich aus den abhängigen Ansprüchen.

Fig. 1 und 5 zeigen gebrauchsfertig verpackte Venenkatheter.

Fig. 2 zeigt einen Katheter mit zurückgezogenem Führungsdraht.

Fig. 3 und 4 zeigen Verschiebevorrichtungen.

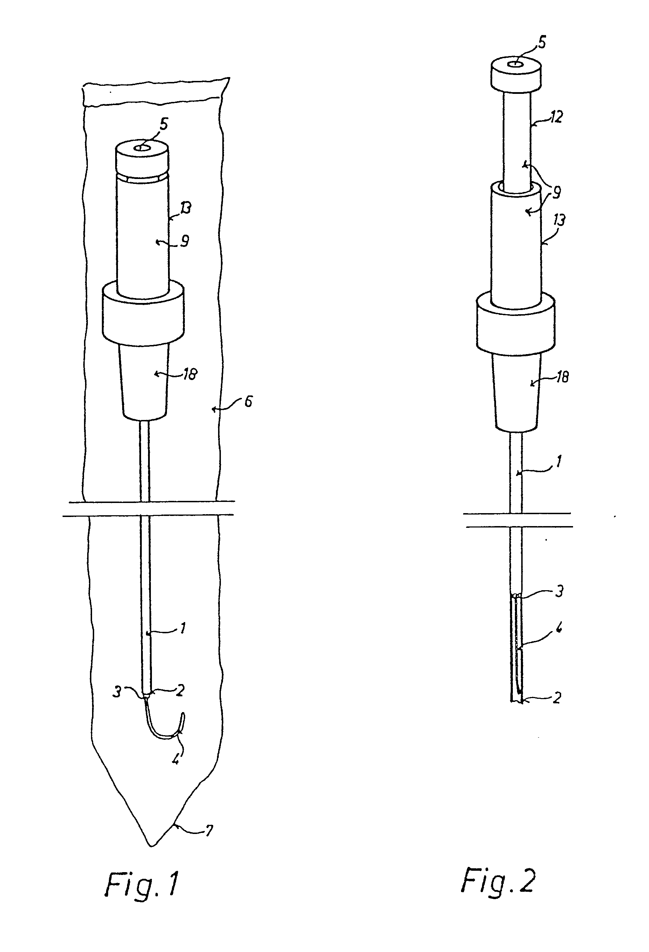

Nachfolgend wird die Erfindung anhand der Fig. 1 bis 5 näher erläutert:

Fig. 1 zeigt vereinfacht eine Ausführungsform des erfindungsgemässen Katheters, verpackt in einer Kunststoffhülle 6, deren distales Ende 7 gewünschtenfalls spitz zulaufend gebildet sein kann, wobei bei der Anwendung des Katheters die Spitze des distalen Endes 7 abgeschnitten werden kann oder mittels der Katheterspitze 2 durchstossbar gestaltet sein kann. Das gekrümmte Ende 4 des Führungsdrahtes 3 ragt aus dem distalen Ende 2 des Katheterschlauches 1 hervor.

Fig. 2 zeigt schematisch den Katheter mit in den Katheterschlauch 1 eingezogenem gekrümmten distalen Ende 4 des Führungsdrahtes 3. Die beiden Hülsen 12, 13 der Verschiebevorrichtung 9 sind hier in eine Endstellung, nämlich die hintere Anschlagstellung, auseinandergezogen.

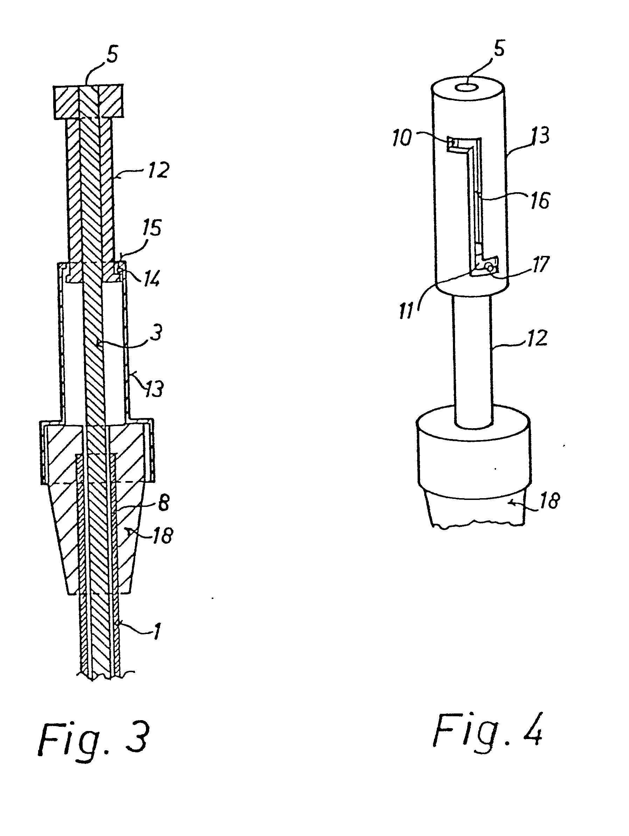

Fig. 3 zeigt einen schematisierten Querschnitt durch eine Ausführungsform der Verschiebevorrichtung 9. Die innere Hülse 12 mit Umfangsrandwulst 14 umschliesst das proximale Ende 5 des Führungsdrahtes 3. Die weibliche Hülse 13 mit einem entsprechenden Innenrandwulst 15 am proximalen Ende 5 ist mit ihrem distalen Ende, das bei dieser Ausführungsform erweitert ist, was aber nicht notwendigerweise so sein muss, lösbar auf das Katheteransatzstück 18 des Katheterschlauches 1 aufgesetzt. Das proximale Ende 8 des Katheterschlauches 1 ist fest mit dem Katheransatzstück 18 verbunden, beispielsweise ist es eingeklebt oder an das Katheteransatzstück angeformt. Nachdem der Katheter über einen peripheren Venenzugang eingeführt und die Katheterspitze 2 ihren Bestimmungsort im zentralen Venensystem erreicht hat, wird die in Fig. 1 angedeutete Kunststoffhülle 6 zusammen mit der Verschiebevorrichtung 9 samt Führungsdraht 3 nach proximal vom Katheteransatzstück 18 getrennt. An das Katheteransatzstück 18 können gewünschtenfalls über ein übliches Zwischenstück mit entsprechenden Aufnahmen, die vorzugsweise konisch sind, sodann beispielsweise eine Spritze, eine Infusionsflasche oder ein Druckmessgerät angeschlossen werden.

Fig. 4 zeigt eine alternative Ausführungsform der Verschiebevorrichtung 9, wobei die äussere Hülse 13 einen axialen Führungsschlitz 16 mit zwei dazu vorzugsweise senkrecht angeordneten endständigen Einrastschlitzen als Anschlageinrichtungen 10, 11 und die innere Hülse 12 einen Anschlagszapfen 17 aufweist.

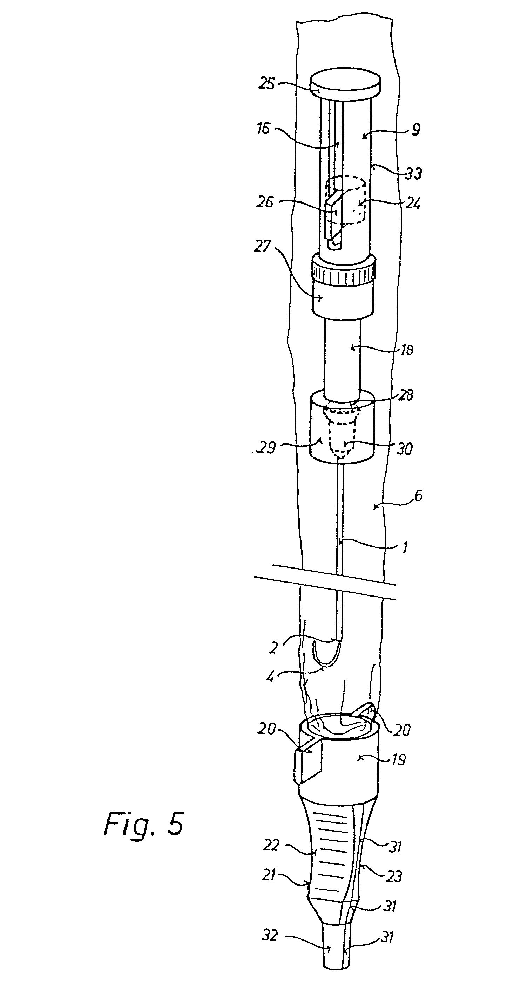

Fig. 5 zeigt ähnlich wie Fig. 1 einen gebrauchsfertig in einer Kunststoffhülle verpackten Venenkatheter. Die Kunststoffhülle 6 endet bei dieser Ausführungsform jedoch in einer Hülse 19. Die Hülse 19, die gewünschtenfalls mit Halteflügeln 20 ausgestattet ist, ist auf ein an sich bekanntes Kathetereinführungsteil 21 aufgesteckt. Das Kathetereinführungsteil 21 besteht aus zwei Teilen 22, 23, die entlang der Trennungslinie 31 voneinander getrennt werden können, sobald die Hülse 19 abgezogen ist. Die Verschiebevorrichtung 9 besteht hier aus einer proximal mit einem Deckel 25 verschlossenen äusseren Hülse 33 mit einem axialen Führungsschlitz 16, in der ein Kolben 24 beweglich angeordnet ist. Der Kolben 24 ist mit dem proximalen Ende 5 des Führungsdrahtes 3 fest verbunden und trägt einen Verschiebeknopf 26. Es versteht sich von selbst, dass die Hülse 33 diametral gegenüber Führungsschlitz 16 einen zweiten Führungsschlitz 16 aufweisen kann, in dem entsprechend ein zweiter Verschiebeknopf 26 des Kolbens 24 geführt wird. Die Verschiebeeinrichtung 9 ist mittels einer als Fortsetzung der Hülse 33 ausgebildeten Überwurfmutter 27 mit Innengewinde mit Katheteransatzstück 18 lösbar verschraubt. Oberhalb des Anschlusskonusses 30 des Katheteranschlussstückes 18, gehalten in einer Umfangsnut 28, befindet sich eine frei drehbare Überwurfmutter 29, mit deren Hilfe der Anschlusskonus 30 des Katheteransatzstückes 18 im Anschlussteil der Venenkanüle (nicht gezeichnet) gesichert werden kann (z.B. Luer-Lock).

Der Führungsdraht 3 füllt das Lumen des Katheterschlauches 1 möglichst weitgehend aus, jedoch nur so weit, dass ein möglichst reibungsarmes Verschieben gegeneinander möglich ist. Zur Verringerung der Reibungskräfte zwischen Katheterschlauch 1 und Führungsdraht 3 können geringe Mengen eines medizinisch annehmbaren Gleitmittels, wie beispielsweise Glycerin oder vorzugsweise Silikonöl, verwendet werden. Die Länge des spazierstockartig gekrümmten Endes 4 des Führungsdrahtes beträgt etwa 1,5 bis 3 cm, vorzugsweise etwa 2 cm. Material, Dimensionen und physikalisches Verhalten des Katheterschlauchs 1 entsprechen dem bei herkömmlichen Kathetern für zentrale Venen. Der Innendurchmesser (Lumen) des Katheterschlauches 1 liegt zwischen 0,5 und 2,0, vorzugsweise 0,8 und 1,6, besonders bevorzugt 1,1 und 1,2 mm. Der Aussendurchmesser des Katheterschlauches 1 liegt zwischen 0,7 und 2,4 mm. Bevorzugte Aussendurchmesser sind 1,4, 1,7 und 2,1 mm. Die Länge des Katheterschlauches 1 sollte etwa 40 bis 80 cm, vorzugsweise etwa 60 cm betragen. Um die Einführung und Lage des Katheterschlauches 1 im Körper röntgenologisch verfolgen zu können, ist es zweckmässig, auf dem Katheterschlauch 1 in axialer Richtung zumindest einen Röntgenkontraststreifen, vorzugsweise mindestens zwei Röntgenkontraststreifen auf den Umfang verteilt, vorzusehen. Um dem Untersucher es auch zu ermöglichen, sich ohne Röntgendarstellung ein Bild von der Lage bzw. Vorschubslänge des Katheterschlauches 1 zu machen, ist es zweckmässig, in anatomisch bedingten und/oder in regelmässigen Abständen Markierungen und/oder Graduierungen anzubringen. Vorteilhaft ist beispielsweise eine Graduierung mit Abständen von 50 mm. Material und physikalisches Verhalten des Führungsdrahtes 3 einschliesslich des gekrümmten distalen Endes 4 entsprechen dem bei herkömmlichen Kathetern für arterielle Zugänge. Der Führungsdraht 3 ist beispielsweise ein kunststoffummantelter Metalldraht, wobei der Metalldraht vorzugsweise bereits vor dem Beginn der Krümmung des spazierstockartig gekrümmten distalen Endes 4 endet, oder ist vollständig als Kunststoffdraht ausgeführt. Die Krümmung des distalen Endes 4 wird auf übliche Weise erzeugt, beispielsweise durch entsprechende Reckung unter Erwärmen. Um die Flexibilität des spazierstockartig gekrümmten distalen Endes 4 zu erhöhen, ist es zweckmässig, dessen Durchmesser auf etwa die Hälfte bis etwa Dreiviertel des Durchmessers des ungekrümmten Teils des Führungsdrahts 3 einzustellen. Diese Massnahme ist ein weiterer Gegenstand der Erfindung. Der Krümmungsradius des distalen Endes 4 liegt etwa zwischen 1 und 10 mm.

Der erfindungsgemässe Katheter ist zweckmässigerweise so ausgestaltet, dass er unter sterilen Kautelen unmittelbar ohne Zuhilfenahme weiterer Mittel angewendet werden kann. In einer besonderen Ausführungsform umfasst der Katheter deshalb ein Punktionsbesteck, das die Punktion der Vene und das Einführen des Katheters erlaubt. Vorteilhaft ist hierbei ein Punktionsbesteck, das die Anwendung der dem Fachmann bekannten Vorgehensweise ermöglicht: Über eine Stahlkanüle ist eine flexible Kunststoffkanüle geschoben, wobei die geschliffene Spitze der Stahlkanüle freibleibt. Nach erfolgter Punktion wird die Stahlkanüle aus der Kunststoffkanüle herausgezogen, während die Kunststoffkanüle in der Vene verbleibt. Der Katheterschlauch wird dann durch die Kunststoffkanüle in die Vene geschoben. Sobald das distale Katheterende die gewünschte Ziellage erreicht hat, kann die Kunststoffkanüle ebenfalls aus der Vene zurückgezogen werden.

Der Katheterschlauch 1 mit in ihm liegendem Führungsdraht 3 wird zweckmässigerweise samt am proximalen Ende 8 angebrachtem Ansatzstück 18 für Spritzen oder Infusionsschläuche steril in einer beidseitig verschlossenen, durchsichtigen Kunststoffschutzhülle 6 zur Verfügung gestellt. Das gekrümmte distale Ende 4 des Führungsdrahtes 3 ragt während der Lagerung des Katheters aus dem distalen Ende 2 des Katheterschlauches 1 heraus. Durch diese Massnahme wird vermieden, dass während der Lagerzeit die Krümmung des gekrümmten Endes 4 des Führungsdrahtes 3 beeinträchtigt wird.

Um den Katheterschlauch 1 bei Gebrauch in die in der Vene sitzende Kanüle einführen zu können, wird das in der Kunststoffschutzhülle 6 aus dem Katheterschlauch 1 herausragende distale gekrümmte Ende 4 des Führungsdrahtes 3 in den Katheterschlauch 1 durch Zug am proximalen Ende 5 des Führungsdrahtes 3 zurückgezogen. Sodann wird bei der Ausführungsform nach Fig. 1 die Kunststoffschutzhülle 6 an ihrem distalen Ende 7 geöffnet und das distale Ende 2 des Katheterschlauchs 1 (Katheterspitze) in die in der Vene sitzende Kanüle eingeführt. Nachdem die Katheterspitze 2 durch die Kunststoffkanüle um einige Zentimeter in die Vene vorgeschoben ist, wird das gekrümmte Ende 4 des Führungsdrahtes 3 wieder so weit aus der Katheterspitze 2 herausgeschoben, dass es seine gekrümmte Form wieder einnehmen kann.

Bei dem Ausführungsbeispiel nach Fig. 5 wird das Kathetereinführungsteil 21 über Anschlusskonus 32 mit dem Anschlussteil der Kunststoffvenenkanüle (nicht gezeichnet) verbunden und das gekrümmte distale Ende 4 des Führungsdrahtes 3 durch Verschieben des Verschiebeknopfes 26 in die hintere Anschlagstellung in die Katheterspitze 2 zurückgezogen. Sodann wird die Katheterspitze 2 durch das Kathetereinführungsteil 21 in die Kunststoffvenenkanüle geschoben. Die Bohrung des Kathetereinführungsteils 21 verjüngt sich in Richtung auf die Venenkanüle trichterartig auf einen inneren Durchmesser, der in etwa dem inneren Durchmesser der Venenkanüle entspricht. Die Gestaltung erleichtert das Einfädeln der Katheterspitze 2 in die Venenkanüle. Nachdem die Katheterspitze 2 aus dem distalen Ende der Venenkanüle heraus einige Zentimeter in der Vene vorgedrungen ist, wird das gekrümmte Ende 4 des Führungsdrahtes 3 durch Verschieben des Verschiebeknopfes 26 nach distal in die vordere Anschlagstellung aus der Katheterspitze 2 herausgeschoben. Der Katheterschlauch 1 kann nunmehr so weit in der Vene vorgeschoben werden, bis die Katheterspitze den gewünschten Ort in der Hohlvene erreicht hat. Nunmehr wird die Hülse 19 der Kunststoffhülle 6 von dem Kathetereinführungsteil 21 abgezogen. Die beiden Teile 22, 23 des Kathetereinführungsteils 21 können nun voneinander getrennt werden und das Kathetereinführungsteil 21 somit entfernt werden. Die Verschiebevorrichtung 9 wird vom Katheteransatzstück 18 getrennt. Die Kunststoffhülle 6 und die Verschiebevorrichtung 9 samt Führungsdraht 3 werden sodann nach proximal abgezogen. Der Anschlusskonus 30 des Katheteransatzstückes 18 kann dann in die Anschlussaufnahme der Venenkanüle gesteckt und mittels der Überwurfmutter 29 gesichert werden. Die Ausführungsform nach Fig. 5 ermöglicht ein besonders kontaminationsarmes Einführen des erfindungsgemässen Katheters.

Die erfindungsgemässe begrenzte Verschiebbarkeit, die ungefähr der Länge des gekrümmten distalen Endes 4 des Führungsdrahtes 3 entspricht, kann durch verschiedene gleichwirkende Massnahmen erreicht werden. Beispielsweise sind Katheterschlauch 1 und Führungsdraht 3 jeweils mit einer von zwei konzentrisch ineinander verschiebbaren Hülsen 12, 13 verbunden, deren Verschiebbarkeitsweg durch Anschläge festgelegt ist. Die innenliegende der beiden Hülsen 12, 13 weist beispielsweise am distalen Ende einen Umfangsrandwulst 14 und die aussenliegende der beiden Hülsen 12, 13 als Gegenanschlag am proximalen Ende einen Innenrandwulst 15 auf. In einer anderen Ausführungsform der Erfindung weist die äussere der beiden Hülsen 12, 13 einen axialen Führungsschlitz 16 mit zwei dazu senkrechten endständigen Einrastschlitzen 10, 11, in dem ein Anschlagszapfen 17 der inneren der beiden Hülsen 12, 13 läuft, auf, womit eine bajonettverschlussartige gegenseitige Fixierung der beiden Hülsen 12, 13 in den beiden Endstellungen erzielt wird. Als gleichwirkend wird auch die Bewirkung der gegenseitigen begrenzten Verschiebung der beiden Hülsen 12, 13 mittels einer oder mehrerer Gewindegänge betrachtet.

Der erfindungsgemässe Katheter wird vorzugsweise zusammen mit einer Venenpunktionskanüle, insbesondere einer Venenkanüle, deren Stahlkanüle von einer Kunststoffkanüle umgeben ist und deren Stahlkanüle nach der Punktion der Vene entfernt werden kann, steril zur Verfügung gestellt. Als gemeinsame Verpackung wird einer sogenannten Peelpackung der Vorzug gegeben.

Der erfindungsgemässe Katheter erlaubt die rasche und komplikationsarme Katheterung zentraler Venen über einen peripheren Venenzugang. Von Vorteil ist dabei auch, dass der Katheter in einer Ausstattung bzw. Verpackung zur Verfügung gestellt werden kann, die seine Anwendung erlaubt, ohne dass die Hände des Untersuchers mit dem Katheter in direkte Berührung gelangen.

Die Ausdrücke «proximal» und «distal» werden im Zusammenhang mit der vorliegenden Erfindung aus der Sicht des Untersuchers gesehen, d.h. ein proximales Teil einer erfindungsgemässen Ausführungsform befindet sich bei der Katheterung näher beim Untersucher als ein distales Teil.

## Patentansprüche

1. Katheter zur Katheterung zentraler Venen über periphere Venenzugänge, umfassend einen Katheterschlauch (1) und einen an seinem distalen Ende (4) spazierstockartig gekrümmten Führungsdraht (3), der gegenüber dem Katheterschlauch (1) verschiebbar ist, dadurch gekennzeichnet, dass eine Vorrichtung (9) vorgesehen ist, die die gegenseitige Verschiebbarkeit von Katheterschlauch (1) gegenüber dem Führungsdraht (3) auf ungefähr die Länge des gekrümmten distalen Endes (4) des Führungsdrahtes (3) zu begrenzen erlaubt, wobei in einer Verschiebeendstellung das gekrümmte distale Ende (4) des Führungsdrahtes (3) aus dem distalen Ende (2) des Katheterschlauches (1) herausragt und in der anderen Verschiebeendstellung das gekrümmte distale Ende (4) des Führungsdrahtes (3) vollständig in das distale Ende (2) des Katheterschlauches (1) hineingezogen ist.

2. Katheter nach Anspruch 1, dadurch gekennzeichnet, dass der Katheterschlauch (1) einen Innendurchmesser von 0,5 bis 2 mm, einen Aussendurchmesser von 0,7 bis 2,4 mm und eine Länge von 400 bis 800 mm aufweist und die Länge des gekrümmten distalen Endes (4) des Führungsdrahtes (3) 15 bis 30 mm beträgt.

3. Katheter nach Anspruch 1 oder 2, dadurch gekennzeichnet, dass der Durchmesser des gekrümmten distalen Endes (4) des Führungsdrahtes (3) geringer ist als der Durchmesser des geraden Teils des Führungsdrahtes (3).

4. Katheter nach Anspruch 3, dadurch gekennzeichnet, dass der Durchmesser des gekrümmten distalen Endes (4) des Führungsdrahtes (3) etwa die Hälfte bis etwa Dreiviertel des Durchmessers des geraden Teils des Führungsdrahtes (3) beträgt.

5. Katheter nach einem der Ansprüche 1 bis 4, dadurch gekennzeichnet, dass die gegenseitige begrenzte Verschiebbarkeit von Katheterschlauch (1) gegenüber dem Führungsdraht (3) durch zwei konzentrische ineinander verschiebbare Hülsen (12, 13), deren eine mit dem Katheterschlauch (1) und deren andere mit dem Führungsdraht (3) verbunden ist.

6. Katheter nach Anspruch 5, dadurch gekennzeichnet, dass die innenliegende der beiden Hülsen (12, 13) als Anschlag einen Umfangsrandwulst (14) und die aussenliegende der beiden Hülsen (12, 13) als Gegenanschlag einen Innenrandwulst (15) aufweist.

7. Katheter nach Anspruch 5, dadurch gekennzeichnet, dass die aussenliegende der beiden Hülsen (12, 13) einen axialen Führungsschlitz (16) mit zwei dazu senkrechten endständigen Einrastschlitzen (10, 11), in dem ein Anschlagszapfen (17) der innenliegenden der beiden Hülsen (12, 13) läuft, aufweist.

8. Katheter nach einem der Ansprüche 1 bis 4, dadurch gekennzeichnet, dass die gegenseitige begrenzte Verschiebbarkeit von Katheterschlauch (1) gegenüber dem Führungsdraht (3) durch eine Hülse (33) mit einem oder mehreren axialen Führungsschlitzen (16), in der ein mit dem Führungsdraht (3) verbundener Kolben (24), der einen oder mehrere in den Führungsschlitzen (16) laufende Verschiebeknöpfe (26) aufweist, läuft, gegeben ist.

9. Katheter nach Anspruch 5, dadurch gekennzeichnet, dass die gegenseitige begrenzte Verschiebbarkeit der beiden Hülsen (12, 13) durch einen oder mehrere Gewindegänge bewerkstelligt wird.

10. Katheter nach einem der Ansprüche 1 bis 9, dadurch gekennzeichnet, dass er von einer Kunststoffhülle (6) umgeben ist.

11. Katheter nach Anspruch 10, dadurch gekennzeichnet, dass das distale Ende (7) der Kunststoffhülle (6) spitz zuläuft.

12. Katheter nach Anspruch 11, dadurch gekennzeichnet, dass das distale Ende (7) der Kunststoffhülle (6) mittels der Katheterspitze (2) durchstossbar ist.

13. Katheter nach Anspruch 10, dadurch gekennzeichnet, dass die Kunststoffhülle (6) am distalen Ende mit einer Hülse (19) verbunden ist, die auf ein Kathetereinführungsteil (21) aufsteckbar ist.

14. Katheter nach einem der Ansprüche 1 bis 13, dadurch gekennzeichnet, dass er zusammen mit einer passenden Venenpunktionskanüle in einer Verpackung zur Verfügung gestellt wird.

15. Katheter nach Anspruch 14, dadurch gekennzeichnet, dass er in einer Peelpackung verpackt ist.

## Claims

1. A catheter for catheterizing central veins via peripheral vein approaches, comprising a catheter tube (1) and a guide wire (3) which is curved in the manner of a walking stick at its distal end (4) and is displaceable relative to the catheter tube (1), characterized in that a device (9) is provided which enables the mutual displaceability of the catheter tube (1) relative to the guide wire (3) to be limited approximately to the length of the curved distal end (4) of the guide wire (3), the curved distal end (4) of the guide wire (3) in one end position of the displacement protruding from the distal end (2) of the catheter tube (1) and, in the other end position of displacement, the curved distal end (4) of the guide wire (3) being completely drawn into the distal end (2) of the catheter tube (1).

2. Catheter according to Claim 1, characterized in that the catheter tube (1) has an internal diameter of 0.5 to 2 mm, an external diameter of 0.7 to 2.4 mm and a length of 400 to 800 mm, and the length of the curved distal end (4) of the guide wire (3) is 15 to 30 mm.

3. Catheter according to Claim 1 or 2, characterized in that the diameter of the curved distal end (4) of the guide wire (3) is smaller than the diameter of the straight portion of the guide wire (3).

4. Catheter according to Claim 3, characterized in that the diameter of the curved distal end (4) of the guide wire (3) is approximately half up to approximately three quarters of the diameter of the straight portion of the guide wire (3).

5. Catheter according to one of Claims 1 to 4, characterized in that the means for the limited mutual displaceability of the catheter tube (1) relative to the guide wire (3) are provided by two concentric telescopic sleeves (12, 13), of which one is joined to the catheter tube (1) and the other is joined to the guide wire (3).

6. Catheter according to Claim 5, characterized in that the internal one of the two sleeves (12, 13) has a peripheral edge bead (14) as a stop and the external one of the two sleeves (12, 13) has an internal edge bead (15) as an abutment.

7. Catheter according to Claim 5, characterized in that the external one of the two sleeves (12, 13) has an axial guide slot (16) with two engagement slots (10, 11) perpendicular thereto at the end, a stop pin (17) of the internal one of the two sleeves (12, 13) running in the guide slot.

8. Catheter according to one of Claims 1 to 4, characterized in that the limited mutual displaceability of the catheter tube (1) relative to the guide wire (3) is provided by a sleeve (33) which has one or more axial guide slots (16) and in which a piston (24) runs which is joined to the guide wire (3) and has one or more displacement knobs (26) running in the guide slots (16).

9. Catheter according to Claim 5, characterized in that the limited mutual displaceability of the two sleeves (12, 13) is accomplished by one or more threaded turns.

10. Catheter according to one of Claims 1 to 9, characterized in that it is surrounded by a plastic cover (6).

11. Catheter according to Claim 10, characterized in that the distal end (7) of the plastic cover (6) is pointed.

12. Catheter according to Claim 11, characterized in that the distal end (7) of the plastic cover (6) is pierceable by the catheter tip (2).

13. Catheter according to Claim 10, characterized in that the distal end of the plastic cover (6) is joined to a sleeve (19) which can be slipped over a catheter insertion part (21).

14. Catheter according to one of Claims 1 to 13, characterized in that it is supplied in one package together with a matching venipuncture cannula.

15. Catheter according to Claim 14, characterized in that it is packaged in a peel pack.

## Revendications

1. Cathéter destiné à la cathétérisation de veines centrales en y accédant par des veines périphériques, comprenant un tube de cathéter (1) et un fil de guidage (3) recourbé en bec de canne à son extrémité distale (4), mobile par rapport au tube de cathéter (1), caractérisé par la présence d'un dispositif (9) qui permet de limiter le déplacement relatif du tube de cathéter (1) par rapport au fil de guidage (3) à peu près à la longueur de l'extrémité distale recourbée (4) du fil de guidage (3), l'extrémité distale recourbée (4) du fil de guidage (3) sortant, dans une position de déplacement extrême, de l'extrémité distale (2) du tube de cathéter (1), tandis que, dans l'autre position de déplacement extrême, l'extrémité distale recourbée (4) du fil de guidage (3) est entièrement inséré dans l'extrémité distale (2) du tube de cathéter (1).

2. Cathéter selon la revendication 1, caractérisé en ce que le tube de cathéter (1) présente un diamètre intérieur de 0,5 à 2 mm, un diamètre extérieur de 0,7 à 2,4 mm et une longueur de 400 à 800 mm, tandis que la longueur de l'extrémité distale recourbée (4) du fil de guidage (3) est de 15 à 30 mm.

3. Cathéter selon la revendication 1 ou 2, caractérisé en ce que le diamètre de l'extrémité distale recourbée (4) du fil de guidage (3) est inférieur au diamètre de la partie rectiligne du fil de guidage (3).

4. Cathéter selon la revendication 3, caractérisé en ce que le diamètre de l'extrémité distale recourbée (4) du fil de guidage (3) est compris entre à peu près la moitié et à peu près les trois quarts du diamètre de la partie rectiligne du fil de guidage (3).

5. Cathéter selon l'une quelconque des revendications 1 à 4, caractérisé en ce que la mobilité relative limitée du tube de cathéter (1) par rapport au fil de guidage (3) est déterminée par deux douilles concentriques mobiles l'une à l'intérieur de l'autre (12, 13), dont l'une est reliée au tube de cathéter (1) et l'autre au fil de guidage (3).

6. Cathéter selon la revendication 5, caractérisé en ce que celle des deux douilles (12, 13) qui est située à l'intérieur comporte un renflement marginal périphérique (14) constituant une butée, tandis que celle des deux douilles (12, 13) qui est située à l'extérieur comporte un renflement marginal intérieur (15), constituant une contre-butée.

7. Cathéter selon la revendication 5, caractérisé en ce que celle des deux douilles (12, 13) qui est située à l'extérieur comporte une fente de guidage axiale (16) avec deux fentes d'encliquetage situées aux extrémités (10, 11) qui lui sont perpendiculaires, dans laquelle se déplace un téton de butée (17) de celle des deux douilles (12, 13) qui est située à l'intérieur.

8. Cathéter selon l'une quelconque des revendications 1 à 4, caractérisé en ce que la mobilité relative limitée du tube de cathéter (1) par rapport au fil de guidage (3) est déterminée par une douille (33) comportant une ou plusieurs fentes de guidage axiales (16), dans laquelle se déplace un piston (24) relié au fil de guidage (3), qui comporte un ou plusieurs boutons de déplacement (26) se déplaçant dans les fentes de guidage (16).

9. Cathéter selon la revendication 5, caractérisé en ce que la mobilité relative limitée des deux douilles (12, 13) est réalisée par un ou plusieurs pas de vis.

10. Cathéter selon l'une quelconque des revendications 1 à 9, caractérisé en ce qu'il est entouré par une gaine (6) en matière plastique.

11. Cathéter selon la revendication 10, caractérisé en ce que l'extrémité distale (7) de la gaine plastique (6) converge coniquement.

12. Cathéter selon la revendication 11, caractérisé en ce que l'extrémité distale (7) de la gaine plastique (6) peut être percée au moyen de la pointe de cathéter (2).

13. Cathéter selon la revendication 10, caractérisé en ce que la gaine plastique (6) est reliée, à l'extrémité distale, à une douille (19) pouvant être emmanchée sur un élément de guidage de cathéter (21).

14. Cathéter selon l'une quelconque des revendications 1 à 13, caractérisé en ce qu'il est fourni, avec une canule de ponction veineuse adaptée, dans un emballage.

15. Cathéter selon la revendication 14, caractérisé en ce qu'il est emballé dans un emballage Peel.

*Fig.1*

*Fig.2*

Fig. 3

Fig. 4

Fig. 5